(19) Europäisches Patentamt
European Patent Office
Office européen des brevets

(11) EP 1 627 626 B1

(12) **FASCICULE DE BREVET EUROPEEN**

(45) Date de publication et mention de la délivrance du brevet:
**08.12.2010 Bulletin 2010/49**

(51) Int Cl.:
***A61K 8/34*** *(2006.01)* ***A61K 8/36*** *(2006.01)*
***A61Q 5/06*** *(2006.01)* ***A61Q 5/10*** *(2006.01)*

(21) Numéro de dépôt: **05291718.4**

(22) Date de dépôt: **11.08.2005**

(54) **Composition tinctoriale comprenant un colorant hydrophobe particulier et un acide**

Färbezusammensetzung enthaltend einen bestimmten hydrophoben Farbstoff und eine Säure

Dyestuff composition comprising a particular hydrophobic colourant and an acid

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **16.08.2004 FR 0451855**

(43) Date de publication de la demande:
**22.02.2006 Bulletin 2006/08**

(73) Titulaire: **L'Oréal**
**75008 Paris (FR)**

(72) Inventeurs:
- **De Boni, Maxime**
  **75020 Paris (FR)**
- **Lagrange, Alain**
  **77700 Coupvray (FR)**

(74) Mandataire: **Fevrier, Murielle Françoise E.**
**L'Oréal**
**D.I.P.I.**
**25-29 Quai Aulagnier**
**92600 Asnières-sur-Seine Cedex (FR)**

(56) Documents cités:
**EP-A- 1 153 599** **DE-A- 4 122 748**
**US-A- 3 816 529** **US-A- 5 891 200**
**US-A1- 2004 045 101** **US-B1- 6 172 242**
**US-B1- 6 206 935**

- **BHAT ET AL.: "Calculated values of the octanol-water partition..." DYES AND PIGMENTS, vol. 52, 2002, pages 145-159, XP004331774**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 627 626 B1

## Description

L'invention a pour objet une composition tinctoriale comprenant dans un milieu approprié un colorant hydrophobe particulier et un acide particulier. L'invention a aussi pour objet l'utilisation de cette composition pour la teinture des fibres kératiniques ainsi que le procédé de teinture mettant en oeuvre cette composition.

Depuis longtemps, de nombreuses personnes cherchent à modifier la couleur de leurs cheveux et en particulier à masquer leurs cheveux blancs.

Pour ce faire, il est connu de teindre les fibres kératiniques de façon permanente par la coloration d'oxydation. Cette technique de coloration consiste à appliquer sur les fibres kératiniques une composition contenant des précurseurs de colorant tels que des bases d'oxydation et des coupleurs. Ces précurseurs, sous l'action d'un agent oxydant, forment dans le cheveu une ou plusieurs espèces colorées.

La variété des molécules mises en jeu au niveau des bases d'oxydation et des coupleurs, permet l'obtention d'une riche palette de couleurs. Les colorations qui en résultent sont permanentes, puissantes, et résistantes aux agents extérieurs, notamment à la lumière, aux intempéries, aux lavages, à la transpiration et aux frottements.

Cependant, la coloration est effectuée à l'aide de produits oxydants tels l'eau oxygénée en milieu basique. Ces agents oxydants attaquent la kératine des cheveux dont les propriétés cosmétiques et mécaniques peuvent se dégrader fortement en cas de colorations répétées ce qui peut entraîner un coiffage ou une mise en forme difficile.

Par ailleurs, il est connu de teindre les fibres kératiniques et en particulier les cheveux humains, avec des compositions de teinture contenant des colorants directs. Les colorants classiques qui sont utilisés sont en particulier des colorants du type nitrés benzèniques, anthraquinoniques, nitropyridiniques, azoïques, xanthéniques, acridiniques, aziniques, triarylméthane ou des colorants naturels. Ces colorants peuvent être non ioniques, anioniques, cationiques ou amphotères. Ces colorants sont des molécules colorées et colorantes ayant une affinité pour les fibres kératiniques.

Les colorants directs sont en général utilisés dans un milieu constitué majoritairement par de l'eau, avec éventuellement une quantité mineure de solvant tel que des alcools. De telles compositions sont par exemple décrites dans les documents EP 1 366 752, EP 1 369 105. Il est aussi connu afin d'améliorer la solubilité dans l'eau des colorants cationiques, dispersés ou solvants par addition d'un tensio-actif anionique comme dans le brevet US 5 593 459. D'autres compositions de coloration des cheveux utilisant des colorants directs sont divulguées dans US 2004/0045101, US 6,206,935, DE 4122748, EP 1153599 et US 5891200.

Ces compositions contenant un ou plusieurs colorants directs sont appliquées sur les fibres kératiniques pendant un temps nécessaire à l'obtention de la coloration désirée, puis rincés.

Les colorations qui en résultent sont des colorations souvent chromatiques qui sont cependant temporaires ou semi-permanentes car la nature des interactions qui lient les colorants directs à la fibre kératinique, et leur désorption de la surface et/ou du coeur de la fibre sont responsables de leur faible puissance tinctoriale et de leur mauvaise tenue aux lavages ou à la transpiration.

Il existe toujours un besoin de développer de nouvelles compositions de teinture directe pour d'obtenir des nuances variées, en particulier dans des nuances pastels et qui présentent une bonne ténacité, notamment aux agents extérieurs tels que la lumière, le shampooing, la sueur. En particulier, il existe un besoin de développer des compositions de coloration permettant d'obtenir des colorations présentant une ténacité proche de la coloration par oxydation sans les inconvénients liés à la présence d'un agent oxydant.

Ce but est atteint avec la présente invention qui a pour objet une composition de coloration comprenant au moins un colorant direct hydrophobe dont le logP est supérieur à 2, au moins un acide organique ou minéral ayant un pKa inférieur à 4,5, dans un milieu de coloration hydroalcoolique contenant au moins 60 % d'eau en poids du poids total de la composition, le pH de la composition étant inférieur à 7.

La composition de l'invention permet d'obtenir des nuances variant du pastel à des colorations intenses. De plus, la coloration obtenue permet d'atteindre voire de dépasser la ténacité de la coloration d'oxydation. Ainsi, la coloration obtenue est très résistante aux agents extérieurs, notamment aux lavages répétés.

Dans le cadre de l'invention, la valeur du logP représente de façon classique le coefficient de partage du colorant entre l'octanol et l'eau. La valeur du logP peut être calculée selon la méthode décrite dans l'article de Meylan et Howard « Atom / Fragment contribution method for estimating octanol-water partition coefficient », J. Pharm. Sci. 84 : 83-92, 1995.. Cette valeur peut aussi être calculée à partir de nombreux logiciels disponibles sur le marché qui détermine la valeur de logP en fonction de la structure d'une molécule. A titre d'exemple, on peut citer le logiciel Epiwin de l'agence de l'environnement des Etats-Unis.

Les colorants directs qui peuvent être utilisés dans la composition de l'invention sont des colorants hydrophobes connus de la technique qui présentent un logP supérieur à 2. A titre d'exemple, on peut citer :

| Colorant | Structure chimique | logP |
|---|---|---|
| Disperse Red 13 | 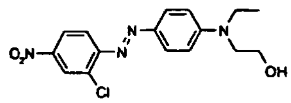 | 5.22 |
| Disperse Green 9 | 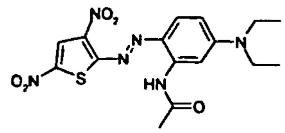 | 4.23 |
| Solvent Black 3 | 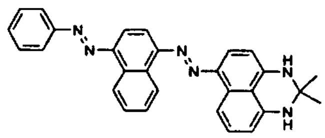 | 7.50 |
| Disperse Blue 148 | 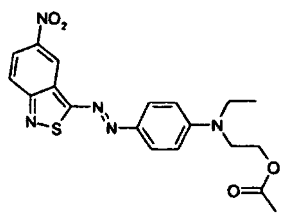 | 4.81 |
| Disperse Violet 63 | 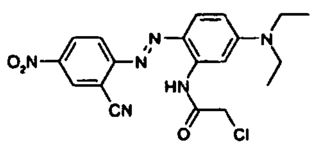 | 5.30 |
| Disperse Blue 60 | 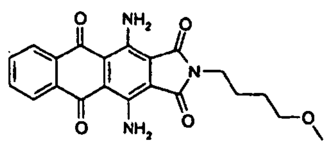 | 3.38 |
| Disperse Blue 14 | 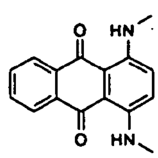 | 4.25 |
| Solvent Orange 15 | 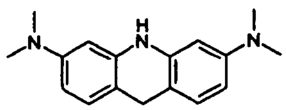 | 3.90 |
| Solvent Orange 7 | 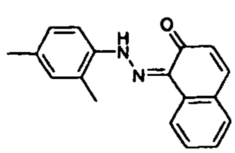 | 4.40 |

(suite)

| Colorant | Structure chimique | logP |
|---|---|---|
| Solvent Blue 14 | 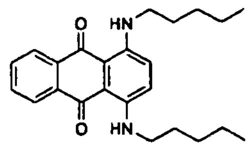 | 8.18 |
| Disperse Yellow 82 | 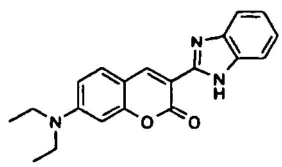 | 3.68 |

Selon un mode de réalisation particulier, le logP du colorant utile dans la composition de l'invention est supérieur à 4.

Le ou les colorants directs présentant un logP supérieur à 2 peuvent être présents dans la composition dans des quantités comprises entre 0,001 à 5 % en poids environ du poids total de la composition.

L'acide organique présentant un pKa inférieur à 4,5 est par exemple choisi parmi l'acide benzoïque, l'acide salicylique, l'acide benzènesulfonique ou leur mélange, de préférence, l'acide benzoïque.

L'acide minéral présentant un pKa inférieur à 4,5 est par exemple choisi parmi l'acide phosphorique, l'acide chlorhydrique, l'acide sulfurique.

La composition de l'invention peut contenir en mélange un ou plusieurs acides organiques ou minéraux utiles pour l'invention.

Selon un mode de réalisation particulier, l'acide est un acide organique.

Selon un mode de réalisation particulier, la composition contient de plus un sel d'acide minéral divalent. Un tel sel d'acide est par exemple le sulfate d'ammonium, l'hydrogénophosphate de sodium, le carbonate de sodium, le sulfate d'ammonium ou leur mélange, de préférence le sulfate d'ammonium.

La quantité d'acide organique, d'acide minéral et de sels d'acide minéral divalent est en général comprise entre 0,001 % et 10 % en poids par rapport au poids total de la composition, de préférence entre 0,05 et 2 %.

Selon un mode de réalisation particulier, la quantité d'eau dans le milieu de coloration hydroalcoolique est comprise entre 60 et 99,5 % en poids du poids total de la composition, de préférence comprise entre 60 et 90 %. Selon un mode de réalisation particulièrement préféré, la quantité en eau est comprise entre 70 et 85 %.

Le milieu de coloration de la composition de l'invention est un mélange hydroalcoolique. A titre d'alcool pouvant être utilisé, on peut notamment citer les alcanols inférieurs en $C_1$-$C_6$, les polyols et éthers de polyols possédant une fonction -OH libre. A titre d'exemple, on peut citer le 2-butoxyéthanol, le propylèneglycol, le monométhyléther de propylèneglycol, le monoéthyléther et le monométhyléther du diéthylèneglycol, l'éthylène glycol monoéthyléther, l'éthylène glycol mono butyl éther, le néopentyl glycol, l'isoprèneglycol ainsi que les alcools aromatiques comme l'alcool benzylique ou le phénoxyéthanol, l'alcool phényléthylique, l'éthanol, l'isopropanol, le n-propanol, le butanol, le n-pentanol, le 1,2-propanediol, le 1,3-propanediol, le 1-méthoxy 2-propanol, le 1-éthoxy 2-propanediol, les 1,3 et 1,4- butanediol, le 1,2-hexanediol ou un mélange de ces alcools.

Selon un mode de réalisation particulier, la quantité d'alcool est au minimum de 5 %, de préférence comprise entre 5 et 35 %. Selon un mode de réalisation préféré, la quantité d'alcool est comprise entre 10 et 25 %.

Les % sont des pourcentages en poids par rapport au poids total de la composition utile dans le procédé de l'invention.

La composition tinctoriale conforme à l'invention peut en outre contenir des colorants directs différents des colorants directs utiles dans la présente invention. Ces colorants directs additionnels sont par exemple des colorants directs nitrés benzéniques neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs benzéniques, on peut citer de manière non limitative les composés suivants

- 1,4-diamino-2-nitrobenzène
- 1-amino-2 nitro-4-p- hydroxyéthylaminobenzène
- 1-amino-2 nitro-4-bis(β-hydroxyéthyl)-aminobenzène

- 1,4-Bis(β-hydroxyéthylamino)-2-nitrobenzéne
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-Diamino-4-nitrobenzène
- 1-amino-2-β-hydroxyéthylamino-5-nitrobenzène
- 1,2-Bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1-β,γ-dihydroxypropyloxy-3-méthylamino-4-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques, on peut citer les colorants azoïques cationiques décrits dans les demandes de brevets WO 95/15144, WO-95/01772 et EP-714954.

Parmi ces composés, on peut tout particulièrement citer les colorants suivants:

- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3$^{e}$ édition :

- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques, on peut citer les colorants suivants :

- Acid Violet 43
- Acid Blue 62
- Basic Blue 22

- Basic Blue 99

ainsi que les composés suivants :

- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques, on peut citer les composés suivants :

- Basic Blue 17
- Basic Red 2.

Parmi les colorants triarylméthaniques, on peut citer les composés suivants :

- Basic Green 1
- Acid blue 9
- Basic Violet 3
- Basic Violet 14
- Basic Blue 7
- Acid Violet 49
- Basic Blue 26
- Acid Blue 7

Parmi les colorants directs naturels utilisables selon l'invention, on peut citer la lawsone, la juglone, l'alizarine, la purpurine, l'acide carminique, l'acide kermésique, la spinulosine. On peut également utiliser les extraits ou décoctions contenant ces colorants naturels et notamment les cataplasmes ou extraits à base de henné.

Lorsque cette composition comprend d'autres colorants directs que ceux présentant un logP supérieur à 2, la composition peut contenir jusqu'à 20 % de colorants directs. Selon ce mode particulier de réalisation, la composition de l'invention peut comprendre une quantité totale de colorants directs comprise entre 0,001 à 10% en poids environ.

La composition de la présente invention peut de plus contenir des bases d'oxydations et des coupleurs classiquement utilisés pour la coloration par oxydation.

A titre d'exemple, on peut citer les paraphénylènediamines, les bis-phénylalkylènediamines, les para-aminophénols, les ortho-aminophénols, les bases hétérocycliques et leurs sels d'addition.

Les coupleurs sont par exemple les coupleurs métaphénylènediamines, les coupleurs méta-aminophénols, les coupleurs métadiphénols, les coupleurs naphtaléniques, les coupleurs hétérocycliques et leurs sels d'addition.

Lorsqu'ils sont présents, les bases et les coupleurs sont chacun généralement présents en quantité comprise entre 0,001 et 10 % en poids environ du poids total de la composition tinctoriale, de préférence entre 0,005 et 6 %.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Ces adjuvants ci dessus sont en général présents en quantité comprise pour chacun d'eux entre 0,01 et 20 % en poids par rapport au poids de la composition.

Pour la teinture des fibres kératiniques humaines, le milieu de coloration est un milieu cosmétique.

Bien entendu, l'homme de l'art veillera à choisir ce ou ces éventuels composés complémentaires de manière telle que les propriétés avantageuses attachées intrinsèquement à la composition conforme à l'invention ne soient pas, ou substantiellement pas, altérées par la ou les adjonctions envisagées.

Le pH de la composition tinctoriale conforme à l'invention qui est inférieur à 7 peut être ajusté à la valeur désirée au moyen d'agents acidifiants ou alcalinisants habituellement utilisés en teinture des fibres kératiniques ou bien encore

à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants, on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante :

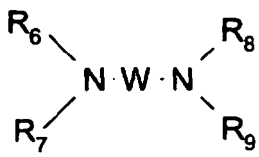

(III)

dans laquelle W est un reste propylène éventuellement substitué par un groupement hydroxyle ou un radical alkyle en $C_1$-$C_4$ ; $R_6$, $R_7$, $R_8$ et $R_9$, identiques ou différents, représentent un atome d'hydrogène, un radical alkyle en $C_1$-$C_4$ ou hydroxyalkyle en $C_1$-$C_4$.

L'invention a aussi pour objet un procédé de teinture directe qui consiste à appliquer sur les fibres kératiniques une composition tinctoriale telle que définie précédemment pendant un temps suffisant pour obtenir la coloration désirée. Le temps de pause est généralement compris entre 1 à 60 minutes environ, de préférence 10 à 60 minutes environ. Après le temps de pause, les fibres kératiniques sont rincées laissant apparaître des fibres colorées.

Selon un mode de réalisation particulier, la température de la composition pendant l'application est inférieure ou égale à 60°C, de préférence entre la température ambiante et 40°C.

Lorsque la composition tinctoriale comprend une base d'oxydation et/ou un coupleur ou lorsqu'on veut effectuer une coloration directe éclaircissante, la composition tinctoriale peut alors contenir un agent oxydant. Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

L'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention. La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Selon un mode de réalisation particulièrement préféré, le pH est inférieur à 7. Il peut être ajusté à la valeur désirée au moyen d'agents acidifiants ou alcalinisants habituellement utilisés en teinture des fibres kératiniques et tels que définis précédemment.

La composition qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

Les exemples qui suivent servent à illustrer l'invention sans toutefois présenter un caractère limitatif.

## EXEMPLES

### Exemple 1

Une composition de coloration est réalisée comme suit :

| | |
|---|---|
| éthanol | 15% |
| alcool benzylique | 5% |
| acide benzoïque | 5 % |
| Disperse red 13 | 0,3% |
| eau | QSP 100 |

La composition est appliquée sur une mèche de cheveux naturels gris contenant 90% de cheveux blancs.
Après une application de 30 minutes à 35°C, les mèches sont rincées et séchées. Les mèches sont colorées en rouge.

## Exemple 2

Une composition de coloration est réalisée comme suit :

| | |
|---|---|
| éthanol | 15% |
| alcool benzylique | 5% |
| acide benzoïque | 0,2% |
| Disperse red 13 | 0.3% |
| eau | QSP 100 |

La composition est appliquée sur une mèche de cheveux naturels gris contenant 90% de cheveux blancs, une mèche de cheveux permanentés gris contenant 90% de cheveux blancs, une mèche de cheveux châtains décolorés.
Après une application de 30 minutes à 40°C, les mèches sont rincées et séchées. Les mèches sont colorées en rouge.
Les mèches colorées sont ensuite soumises à 10 shampooings selon un cycle qui comprend le mouillage des mèches à l'eau, le lavage aux shampooings, un rinçage à l'eau suivi d'un séchage.
La couleur des mèches, avant coloration, après coloration et après les 10 lavages a été évaluée dans le système L* a* b*, au moyen d'un spectrophotomètre CM 2002 MINOLTA ®, (Illuminant D65). Dans ce système L* a* b*, les trois paramètres désignent respectivement l'intensité (L*), a* indique l'axe de couleur vert/rouge et b* l'axe de couleur bleu/jaune. Plus la valeur de L est faible, plus la couleur est foncée ou très intense.
La montée de la coloration est mesurée par (ΔE) selon l'équation suivante :

$$\Delta E = \sqrt{(L^* - L_o{}^*)^2 + (a^* - a_o{}^*)^2 + (b^* - b_o{}^*)^2}$$

Dans cette équation, L*, a* et b* représentent les valeurs mesurées après coloration et L0*, a0* et b0* représentent les valeurs mesurées avant coloration.
La variation de la couleur avant et après 10 lavages est mesurée par (ΔEc) selon l'équation suivante :

$$\Delta Ec = \sqrt{(L_1{}^* - L^*)^2 + (a_1{}^* - a^*)^2 + (b_1{}^* - b^*)^2}$$

Dans cette équation, L*, a* et b* représentent les valeurs mesurées après coloration et L1*, a1* et b1* représentent les valeurs mesurées après 10 lavages.
Le % de dégradation de la couleur par le lavage est évaluée par

$$(\Delta Ec/\Delta E)^*100$$

Les résultats sont reportés dans le tableau 1 ci-dessous.

## EXemple 3 (comparatif)

Une coloration des trois types de mèches décrits ci-dessus a été effectuée à partir de la composition de teinture suivante

| | |
|---|---|
| Hydroxyéthylcellulose (PM 720000) | 0,72 % |
| décylglucoside | 5 % |
| Alcool benzylique | 4% |
| polyéthylèneglycol | 4 % |
| Basic red 51 | 0.2 % |
| eau | QSP 100 |

Après 30 min de pause à température ambiante, les mèches obtenues sont colorées en rouge.

La mesure du % de dégradation est réalisée selon les conditions décrites dans l'exemple 3.

Les résultats sont reportés dans le tableau 1 ci-dessous.

**TABLEAU 1**

| % dégradation de la coloration | Exemple 2 | Exemple 3 |
|---|---|---|
| Cheveux gris naturels | 27 % | 42 % |
| Cheveux gris permanentés | 7 % | 65 % |
| Cheveux décolorés | 7 % | 85 % |

Ces résultats montrent que la composition de l'invention présente une ténacité aux lavages bien supérieure à celle obtenue avec une coloration directe conventionnelle.

## Revendications

1. Composition de coloration comprenant au moins un colorant direct hydrophobe dont le logP est supérieur à 2, au moins un acide organique ou minéral présentant un pKa inférieur à 4,5, dans un milieu de coloration hydroalcoolique contenant au moins 60 % d'eau, le pH de la composition étant inférieur à 7.

2. Composition selon la revendication 1 dans laquelle au moins un des colorants directs hydrophobes présente un logP supérieur à 4.

3. Composition selon la revendication 1 ou 2 dans laquelle l'alcool présent dans la composition est choisi parmi les alcanols inférieurs en $C_1$-$C_6$, les polyols et éthers de polyols, seuls ou en mélange.

4. Composition selon la revendication 3 dans laquelle l'alcool est choisi parmi le 2-butoxyéthanol, le propylèneglycol, le monométhyléther de propylèneglycol, le monoéthyléther et le monométhyléther du diéthylèneglycol, l'éthylène glycol monobutyl éther, l'éthylène glycol monoéthyléther, le néopentyl glycol, l'isoprène glycol, l'éthanol, l'isopropanol, le n-propanol, le butanol, le n-pentanol, le 1,2-propanediol, le 1,3-propanediol, le 1-methoxy 2-propanol, le 1-ethoxy 2-propanediol, le 1,3 et 1,4- butanediol, le 1,2-hexanediol, l'alcool benzylique, le phénoxyéthanol, l'alcool phényléthylique ou un mélange de ces alcools.

5. Composition selon l'une quelconque des revendications 1 à 4 dans laquelle la quantité d'eau dans la milieu de coloration hydroalcoolique est comprise entre 60 et 99,5 % en poids du poids total de la composition.

6. Composition selon la revendication 5 dans laquelle la quantité d'eau est comprise entre 60 et 90 %.

7. Composition selon la revendication 6 dans laquelle la quantité d'eau est comprise entre 70 et 85 %.

8. Composition selon l'une quelconque des revendications précédentes dans laquelle la quantité d'alcool dans le milieu de coloration hydroalcoolique est supérieure à 5 %.

**9.** Composition selon la revendication 8 dans laquelle la quantité d'alcool est comprise entre 5 et 35 %.

**10.** Composition selon la revendication 9 dans laquelle la quantité en alcool est comprise entre 10 et 25 %.

**11.** Composition selon l'une quelconque des revendications précédentes comprenant de plus un sel d'acide minéral divalent.

**12.** Composition selon l'une quelconque des revendications 1 à 11 dans laquelle le ou les colorants directs hydrophobes présentant un logP supérieur à 2 sont présents en quantité comprise entre 0,001 à 5 % en poids environ du poids total de la composition.

**13.** Composition selon l'une quelconque des revendications 1 à 12 dans laquelle le ou les acides organiques sont choisis parmi l'acide benzoïque, l'acide salicylique, l'acide benzènesulfonique, ou leurs mélanges.

**14.** Composition selon l'une quelconque des revendications 1 à 13 dans laquelle l'acide minéral est choisi parmi l'acide phosphorique, l'acide chlorhydrique, l'acide sulfurique.

**15.** Composition selon la revendication 13 dans laquelle la quantité d'acide organique est comprise entre 0,001 et 10 %.

**16.** Composition selon la revendication 15 dans laquelle la quantité d'acide organique est comprise entre 0,05 et 2 %.

**17.** Composition selon l'une quelconque des revendications 11 à 16 dans laquelle le sel d'acide minéral divalent est choisi parmi le sulfate d'ammonium, l'hydrogénophosphate de sodium, le carbonate de sodium ou leurs mélanges.

**18.** Composition selon la revendication 17 dans laquelle la quantité de sels d'acide minéral divalent est comprise entre 0,001 et 10 %.

**19.** Composition selon la revendication 18 dans laquelle la quantité de sels d'acide minéral divalent est comprise entre 0,05 et 2 %.

**20.** Composition selon l'une quelconque des revendications comprenant un ou plusieurs colorants directs additionnels choisis parmi les colorants directs nitrés benzéniques neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

**21.** Composition selon l'une quelconque des revendications précédentes comprenant une base d'oxydation choisie parmi les paraphénylènediamines, les bis-phénylalkylènediamines, les para-aminophénols, les ortho-aminophénols, les bases hétérocycliques et leurs sels d'addition.

**22.** Composition selon l'une quelconque des revendications précédentes comprenant un coupleur choisi parmi les métaphénylènediamines, les méta-aminophénols, les métadiphénols, les coupleurs naphtaléniques, les coupleurs hétérocycliques et leurs sels d'addition.

**23.** Composition selon la revendication 21 dans laquelle la quantité de bases d'oxydation est comprise entre 0,001 à 10 % en poids environ du poids total de la composition tinctoriale.

**24.** Composition selon la revendication 22 dans laquelle la quantité de chacun des coupleurs est comprise entre 0,001 et 10 % en poids environ du poids total de la composition tinctoriale.

**25.** Composition selon l'une quelconque des revendications 1 à 24 comprenant un ou plusieurs adjuvants choisis parmi les agents tensio-actifs anioniques, cationiques, non-ioniques, amphotères, zwitterioniques ou leurs mélanges, les polymères anioniques, cationiques, non-ioniques, amphotères, zwitterioniques ou leurs mélanges, les agents épaississants minéraux ou organiques, les agents épaississants associatifs polymères anioniques, cationiques, non ioniques et amphotères, les agents antioxydants, les agents de pénétration, les agents séquestrants, les parfums, les tampons, les agents dispersants, les agents de conditionnement, les agents filmogènes, les céramides, les agents conservateurs, les agents opacifiants.

**26.** Composition selon la revendication 25 dans laquelle les adjuvants sont présents en quantité comprise pour chacun d'eux entre 0,01 et 20 % en poids par rapport au poids de la composition.

**27.** Procédé de coloration qui consiste à appliquer sur les fibres kératiniques une composition tinctoriale telle que définie selon l'une quelconque des revendications 1 à 26 pendant un temps suffisant pour obtenir la coloration désirée.

**28.** Procédé selon la revendication 27 dans lequel l'application est effectuée à une température inférieure ou égale à 60°C.

**29.** Procédé selon la revendication 28 dans lequel la température est comprise entre la température ambiante et 40°C.

**30.** Utilisation de la composition définie aux revendications 1 à 26 pour la coloration des fibres kératiniques.

**31.** Utilisation selon la revendication 30 pour augmenter la ténacité de la coloration des fibres kératiniques.

**Claims**

**1.** Dye composition comprising at least one hydrophobic direct dye with a logP of greater than 2 and at least one organic or mineral acid with a pKa of less than 4.5, in an aqueous-alcoholic dyeing medium containing at least 60% water, the pH of the composition being less than 7.

**2.** Composition according to Claim 1, in which at least one of the hydrophobic direct dyes has a logP of greater than 4.

**3.** Composition according to Claim 1 or 2, in which the alcohol present in the composition is chosen from $C_1$-$C_6$ lower alkanols, polyols and polyol ethers, alone or as a mixture.

**4.** Composition according to Claim 3, in which the alcohol is chosen from 2-butoxyethanol, propylene glycol, propylene glycol monomethyl ether, diethylene glycol monomethyl ether and monoethyl ether, ethylene glycol monobutyl ether, ethylene glycol monoethyl ether, neopentyl glycol, isoprene glycol, ethanol, isopropanol, n-propanol, butanol, n-pentanol, 1,2-propanediol, 1,3-propanediol, 1-methoxy-2-propanol, 1-ethoxy-2-propanediol, 1,3-and 1,4-butanediol, 1,2-hexanediol, benzyl alcohol, phenoxyethanol and phenylethyl alcohol, or a mixture of these alcohols.

**5.** Composition according to any one of Claims 1 to 4, in which the amount of water in the aqueous-alcoholic dyeing medium is between 60% and 99.5% by weight relative to the total weight of the composition.

**6.** Composition according to Claim 5, in which the amount of water is between 60% and 90%.

**7.** Composition according to Claim 6, in which the amount of water is between 70% and 85%.

**8.** Composition according to any one of the preceding claims, in which the amount of alcohol in the aqueous-alcoholic dyeing medium is greater than 5%.

**9.** Composition according to Claim 8, in which the amount of alcohol is between 5% and 35%.

**10.** Composition according to Claim 9, in which the amount of alcohol is between 10% and 25%.

**11.** Composition according to any one of the preceding claims further comprising a divalent mineral acid salt.

**12.** Composition according to any one of Claims 1 to 11, in which the hydrophobic direct dye(s) with a logP of greater than 2 is (are) present in an amount of between 0.001% and 5% by weight approximately relative to the total weight of the composition.

**13.** Composition according to any one of Claims 1 to 12, in which the organic acid(s) is (are) chosen from benzoic acid, salicylic acid and benzenesulfonic acid, or mixtures thereof.

**14.** Composition according to any one of Claims 1 to 13, in which the mineral acid is chosen from phosphoric acid, hydrochloric acid and sulfuric acid.

**15.** Composition according to Claim 13, in which the amount of organic acid is between 0.001% and 10%.

**16.** Composition according to Claim 15, in which the amount of organic acid is between 0.05% and 2%.

**17.** Composition according to any one of Claims 11 to 16, in which the divalent mineral acid salt is chosen from ammonium sulfate, sodium hydrogen phosphate and sodium carbonate, or mixtures thereof.

**18.** Composition according to Claim 17, in which the amount of divalent mineral acid salts is between 0.001% and 10%.

**19.** Composition according to Claim 18, in which the amount of divalent mineral acid salts is between 0.05% and 2%.

**20.** Composition according to any one of the claims, comprising one or more additional direct dyes chosen from neutral, acidic or cationic nitrobenzene direct dyes, neutral, acidic or cationic azo direct dyes, neutral, acidic or cationic quinone and in particular anthraquinone direct dyes, azine direct dyes, triarylmethane direct dyes, indoamine direct dyes and natural direct dyes.

**21.** Composition according to any one of the preceding claims, comprising an oxidation base chosen from para-phenylenediamines, bis(phenyl)alkylenediamines, para-aminophenols, ortho-aminophenols and heterocyclic bases, and the addition salts thereof.

**22.** Composition according to any one of the preceding claims, comprising a coupler chosen from metaphenylenediamines, meta-aminophenols, metadiphenols, naphthalene-based couplers and heterocyclic couplers, and the addition salts thereof.

**23.** Composition according to Claim 21, in which the amount of oxidation bases is between 0.001% and 10% by weight approximately relative to the total weight of the dye composition.

**24.** Composition according to Claim 22, in which the amount of each of the couplers is between 0.001% and 10% by weight approximately relative to the total weight of the dye composition.

**25.** Composition according to any one of Claims 1 to 24, comprising one or more adjuvants chosen from anionic, cationic, nonionic, amphoteric or zwitterionic surfactants or mixtures thereof, anionic, cationic, nonionic, amphoteric or zwitterionic polymers or mixtures thereof, mineral or organic thickeners, anionic, cationic, nonionic and amphoteric polymeric associative thickeners, antioxidants, penetrants, sequestrants, fragrances, buffers, dispersants, conditioning agents, film-forming agents, ceramides, preserving agents and opacifiers.

**26.** Composition according to Claim 25, in which the adjuvants are present in an amount for each of them of between 0.01% and 20% by weight relative to the weight of the composition.

**27.** Dyeing process, which consists in applying to keratin fibres a dye composition as defined according to any one of Claims 1 to 26, for a time that is sufficient to obtain the desired coloration.

**28.** Process according to Claim 27, in which the application is performed at a temperature of less than or equal to 60°C.

**29.** Process according to Claim 28, in which the temperature is between room temperature and 40°C.

**30.** Use of the composition defined in Claims 1 to 26 for dyeing keratin fibres.

**31.** Use according to Claim 30 for increasing the colour-fastness of keratin fibres.

## Patentansprüche

**1.** Zusammensetzung zum Färben, die mindestens einen hydrophoben Direktfarbstoff, dessen logP über 2 liegt, und mindestens eine organische oder anorganische Säure, die einen pKa-Wert unter 4,5 aufweist, in einem wässrigalkoholischen Medium zum Färben enthält, das mindestens 60 % Wasser enthält, wobei der pH-Wert der Zusammensetzung unter 7 liegt.

2. Zusammensetzung nach Anspruch 1, bei der mindestens einer der hydrophoben Direktfarbstoffe einen logP über 4 aufweist.

3. Zusammensetzung nach Anspruch 1 oder 2, bei der der in der Zusammensetzung enthaltene Alkohol einzeln oder in Form von Gemischen unter den niederen Ci-s-Alkoholen, Polyolen und Polyolethern ausgewählt ist.

4. Zusammensetzung nach Anspruch 3, bei der der Alkohol unter 2-Butoxyethanol, Propylenglycol, Propylenglycolmonomethylether, Diethylenglycolmonomethylether, Diethylenglycolmonoethylether, Ethylenglycolmonobutylether, Ethylenglycolmonoethylether, Neopentylglycol, Isoprenglycol, Ethanol, Isopropanol, n-Propanol, Butanol, n-Pentanol, 1,2-Propandiol, 1,3-Propandiol, 1-Methoxy-2-propanol, 1-Ethoxy-2-propandiol, 1,3-Butandiol, 1,4-Butandiol, 1,2-Hexandiol, Benzylalkohol, Phenoxyethanol, Phenylethylalkohol oder einem Gemisch dieser Alkohole ausgewählt ist.

5. Zusammensetzung nach einem der Ansprüche 1 bis 4, bei der die Wassermenge in dem wässrig-alkoholischen Medium zum Färben im Bereich von 60 bis 99,5 Gew.-% des Gesamtgewichts der Zusammensetzung liegt.

6. Zusammensetzung nach Anspruch 5, wobei die Wassermenge im Bereich von 60 bis 90 Gew.-% liegt.

7. Zusammensetzung nach Anspruch 6, wobei die Wassermenge im Bereich von 70 bis 85 % liegt.

8. Zusammensetzung nach einem der vorhergehenden Ansprüche, bei der die Menge des Alkohols in dem wässrig-alkoholischen Medium zum Färben größer als 5 % ist.

9. Zusammensetzung nach Anspruch 8, wobei die Menge des Alkohols im Bereich von 5 bis 35 % liegt.

10. Zusammensetzung nach Anspruch 9, wobei die Menge des Alkohols im Bereich von 10 bis 25 % liegt.

11. Zusammensetzung nach einem der vorhergehenden Ansprüche, die ferner ein Salz einer zweiwertigen anorganischen Säure enthält.

12. Zusammensetzung nach einem der Ansprüche 1 bis 11, bei der der oder die hydrophoben Direktfarbstoffe, die einen logP über 2 aufweisen, in einem Mengenanteil im Bereich von etwa 0,001 bis 5 Gew.-% des Gesamtgewichts der Zusammensetzung enthalten sind.

13. Zusammensetzung nach einem der Ansprüche 1 bis 12, wobei die organische(n) Säure(n) unter Benzoesäure, Salicylsäure, Benzolsulfonsäure oder deren Gemischen ausgewählt ist.

14. Zusammensetzung nach einem der Ansprüche 1 bis 13, bei de die anorganische Säure unter Phosphorsäure, Salzsäure und Schwefelsäure ausgewählt ist.

15. Zusammensetzung nach Anspruch 13, wobei der Mengenanteil der organischen Säure im Bereich von 0,001 bis 10 % liegt.

16. Zusammensetzung nach Anspruch 15, wobei der Mengenanteil der organischen Säure im Bereich von 0,05 bis 2 % liegt.

17. Zusammensetzung nach einem der Ansprüche 11 bis 16, wobei das Salz einer zweiwertigen anorganischen Säure unter Ammoniumsulfat, Natriumhydrogensulfat, Natriumcarbonat oder deren Gemischen ausgewählt ist.

18. Zusammensetzung nach Anspruch 17, wobei der Mengenanteil der Salze einer zweiwertigen anorganischen Säure im Bereich von 0,001 bis 10 % liegt.

19. Zusammensetzung nach Anspruch 18, wobei der Mengenanteil der Salze einer zweiwertigen anorganischen Säure im Bereich von 0,05 bis 2 % liegt.

20. Zusammensetzung nach einem der vorhergehenden Ansprüche, die einen oder mehrere ergänzende Direktfarbstoffe enthält, die unter den neutralen, sauren oder kationischen nitrierten direktziehenden Benzolfarbstoffen, neutralen, sauren oder kationischen direktziehenden Azofarbstoffen, neutralen, sauren oder kationischen direktziehenden Chinonfarbstoffen und insbesondere Anthrachinon-Farbstoffen, direktziehenden Azinfarbstoffen, direktziehenden Triarylmethanfarbstoffen, direktziehenden Indoaminfarbstoffen und direktziehenden natürlichen Farbstoffen ausgewählt sind.

**21.** Zusammensetzung nach einem der vorhergehenden Ansprüche, die eine Oxidationsbase enthält, die unter den para-Phenylendiaminen, Bisphenylalkylendiaminen, para-Aminophenolen, ortho-Aminophenolen, heterocyclischen Basen und ihren Additionssalzen ausgewählt ist.

**22.** Zusammensetzung nach einem der vorhergehenden Ansprüche, die eine Kuppler enthält, der unter den meta-Phenylendiaminen, meta-Aminophenolen, meta-Dihydroxybenzolen, Naphthalinkupplern und heterocyclischen Kupplern und ihren Additionssalzen ausgewählt ist.

**23.** Zusammensetzung nach Anspruch 21, wobei der Mengenanteil der Oxidationsbasen im Bereich von etwa 0,001 bis 10 Gew.-% des Gesamtgewichts der Farbmittelzusammensetzung liegt.

**24.** Zusammensetzung nach Anspruch 22, wobei der Mengenanteil jedes Kupplers im Bereich von etwa 0,001 bis 10 Gew.-% des Gesamtgewichts der Farbmittelzusammensetzung liegt.

**25.** Zusammensetzung nach einem der Ansprüche 1 bis 24, die einen oder mehrere Zusatzstoff enthält, die unter den anionischen, kationischen, nichtionischen, amphoteren, zwitterionischen grenzflächenaktiven Stoffen oder deren Gemischen, anionischen, kationischen, nichtionischen, amphoteren, zwitterionischen Polymeren oder deren Gemischen, anorganischen oder organischen Verdickungsmitteln, anionischen, kationischen, nichtionischen und amphoteren, polymeren assoziativen Verdickungsmitteln, Antioxidantien, Penetrationsmitteln, Maskierungsmitteln, Parfums, Puffern, Dispergiermitteln, Konditioniermitteln, Filmbildnern, Ceramiden, Konservierungsmitteln und Trübungsmitteln ausgewählt ist.

**26.** Zusammensetzung nach Anspruch 25, bei der die Zusatzstoffe jeder in einem Mengenanteil von 0,01 bis 20 Gew.-%, bezogen auf das Gesamtgewicht der Zusammensetzung, enthalten sind.

**27.** Verfahren zum Färben, das darin besteht, eine Farbmittelzusammensetzung, wie sie in einem der Ansprüche 1 bis 26 definiert ist, während einer Zeitspanne, die ausreichend ist, um die gewünschte Färbung zu bilden, auf die Keratinfasern aufzubringen.

**28.** Verfahren nach Anspruch 27, bei dem die Anwendung bei einer Temperatur von kleiner oder gleich 60˚C erfolgt.

**29.** Verfahren nach Anspruch 28, wobei die Temperatur im Bereich von Raumtemperatur bis 40˚C liegt.

**30.** Verwendung der in den Ansprüchen 1 bis 26 definierten Zusammensetzung zum Färben von Keratinfasern.

**31.** Verwendung nach Anspruch 30, um die Beständigkeit der Färbung der Keratinfasern zu verbessern.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1366752 A
- EP 1369105 A
- US 5593459 A
- US 20040045101 A
- US 6206935 B
- DE 4122748
- EP 1153599 A
- US 5891200 A
- WO 9515144 A
- WO 9501772 A
- EP 714954 A


### Littérature non-brevet citée dans la description


- **Meylan ; Howard.** Atom / Fragment contribution method for estimating octanol-water partition coefficient. *J. Pharm. Sci.,* 1995, vol. 84, 83-92